# EUROPEAN PATENT APPLICATION

(11) **EP 2 208 788 A1**
(43) Date of publication of application: **21.07.2010**
(21) Application number: 08832492.6
(22) Date of filing: 19.09.2008
(51) Int. Cl.: C12N 15/29, A01H 5/00

(54) **MANIPULATION OF THE FUNCTION OF AtDBP1 IN ORDER TO GENERATE POTYVIRUS RESISTANCE**

(30) Priority: 21.09.2007 ES 200702498
(71) Applicant: Consejo Superior De Investigaciones Científicas, 28006 Madrid (ES); Universidad Politecnia de Valencia, 46022 Valencia (ES)
(72) Inventor: CARRASCO JIMÉNEZ, José Luis, E-46019 Valencia (ES); CASTELLÓ LLOPIS, María José, E-46006 Valencia (ES); VERA VERA, Pablo, E-46530 Puzol Valencia (ES)
(74) Representative: Pons Ariño, Angel
(86) International application number: PCT/ES2008/000595
(87) International publication number: WO 2009/037369

(57) **Abstract**

**MANIPULATION OF THE FUNCTION OF AtDBP1 IN ORDER TO GENERATE POTYVIRUS RESISTANCE**

The invention relates to the use of a mutation for the generation of virus-resistant mutant plants and, specifically, to the use of mutation 8.1 of the AtDBP1 gene of Arabidopsis thalianain order to modify the phenotype of the plant as a regulator of plant potyvirus resistance, as well as to the resulting genetically modified plants having greater potyvirus infection resistance than unmodified plants.

SEQUENCE LISTING

<110> Polytechnic University of Valencia and Higher Centre for Scientific Research
<120> MANIPULATION OF THE FUNCTION OF AtDBP1 IN ORDER TO GENERATE POTYVIRUS
RESISTANCE
<130> AX089151
<150> P200702498
<151> 2007-09-21
<160> 10
<170> PatentIn version 3.3

<210> 1
<211> 22
<212> DNA
<213> Artificial
<220>
<223> Direct sequence of the primer for the AtDBP1 gene
<400> 1
gtctgagttt gttcctgcta cg

<210> 2
<211> 21
<212> DNA
<213> Artificial
<220>
<223> Reverse sequence of the primer for the AtDBP1 gene
<400> 2
tactgctcat gggtttgtgg t

<210> 3
<211> 24
<212> DNA
<213> Artificial
<220>
<223> Direct sequence of the eIF(iso)4e primer
<400> 3
cgtctcagaa gaaaactcaa ctgc

<210> 4
<211> 23
<212> DNA
<213> Artificial
<220>
<223> Reverse sequence of the eIF(iso)4e primer
<400> 4
catccttcctc tggcttcaca ctc

<210> 5
<211> 20
<212> DNA
<213> Artificial
<220>
<223> Direct sequence of the primer for the eEF1-alpha gene
<400> 5
gcacagtcat tgatgcccca

<210> 6
<211> 21
<212> DNA
<213> Artificial
<220>
<223> Reverse sequence of the primer for the eEF1-alpha gene
<400> 6
cctcaagaag agttggtccc t

<210> 7
<211> 20
<212> DNA

<213> Artificial
<220>
<223> Direct sequence of the primer for the GFP gene
<400> 7
acgtaaacgg ccacaagttc

<210> 8
<211> 20
<212> DNA
<213> Artificial
<220>
<223> Reverse sequence of the primer for the GFP gene
<400> 8
aagtcgtgct gcttcatgtg

<210> 9
<211> 2303
<212> DNA
<213> Artificial
<220>
<223> Nucleotide sequence of the AtDBP1 gene (At2g25620)
<400> 9
gccggcgaac agttttatta tctctctctt tctgcgtgtt ccaatttctt ccacaaaacc 60
ctaatttctt cttaaaccct gaaattcgtg taacctttca acaatctctt caattttgtt 120
ttcgattctg aatccattgt ttacgaatct ctcatctaaa gctacattct tcgttaaatc 180
gtttttaatc gactcgctca ctcgtgagtt gactcgtcag ctgtttccat aattgcttat 240
tgaattttta tacgatgatg tttcaatatg gaagaaacta gaggaatttc tgatccagag 300
aatgggagtt cgagttacgg cggtaaaccg ccgaatccac tctccttctc ttcttcctcc 360
gccgctgctg ccgtttacag gcaaaccttc gacggcgagc gatcgttggc gccgtgtaat 420
aagaggtcac tggttcgaca ctcatctctc gtaagattcg tttctcttca acttgatttg 480
tggatcagtt aggtttattt tgtgtattga atgatcaatt taagttgagg ttttgtaatt 540
attgctctgc ttttggattt gatcattatt ggtttcctaa tgtgtataat taaggtagaa 600
agtttctgtt tttaatgtgt cattatgaga tgtttgaggt tttctgattg attttcattt 660
tggtaggtga agacaatggt gtcagatata tctgttgaga atgagtttac tatagagaag 720
aacaagtctg agtttgttcc tgctacgcgt tctggagctt ggtctgatat tggctccagg 780
tcaagcatgg aagatgctta tctatgcgtt gataatttca tggatagctt tggccttctg 840
aattctgagg ctggtccaag tgccttctat ggggtatgtt ctttcttcag ttttcctgat 900
tttttgtaga tttgtagcat aagcaaacaa ctgtgaatgt agtgaaaata tggggatctc 960
ttattgaatt ttgtttgtta ttaggtattt gatggacatg gtgggaagca tgccgctgag 1020
tttgcatgtc accatatacc gaggtacatt gttgaagatc aagagtttcc tagtgaaatc 1080
aataaggtgt tatcttcagc atttcttcaa acagacactg ccttcttaga ggcgtgttca 1140
ttggatggga gccttgcttc aggaactact gctttggcag ctattctttt tggaaggttt 1200
gttgattcaa ctcttattat cttttagttt tgtctaacct aaactcttac atcttggtta 1260
tgttaaagaa aaacgtgttt gggaatagat gcttatcctc aactgcgtag tgtgcataat 1320
cttgaccttt aacacaatca tgtacaacta attcgcctgt agatgtattc tctgcccata 1380
tgtgctgctg ctataatgta gttattgtct catatggtct ttgagtcacg tatatacttg 1440
ctcatttcat actctattag tattctggta tgaaaccgtt tggtaccgtg ttaacatata 1500
gttttgtggt aacaggtcgt tggtggtagc aaacgctgga gattgcagag cagtcttatc 1560
ccgtcaggga aaagccattg aaatgtcaag agaccacaaa cccatgagca gtaaggaaag 1620
gagacgcatt gaagcatcgg gtggacatgt attcgatggc tatctaaatg gacaacttaa 1680
tgtggctcga gcgctaggtg actttcatat ggaaggcatg aagaagaaga aagatggttc 1740
tgattgtgga cctctaattg cagagcctga gctcatgaca acaaaactaa cagaagagga 1800
tgagttcctt ataattgggt gtgacggggt ttgggatgtg ttcatgagcc agaatgctgt 1860
agattttgcc agaaggagac tacaggagca caatgacccg gtcatgtgta gtaaggagct 1920
ggttgaggaa gctttgaaga ggaagagtgc tgataatgtg acggcagtgg ttgtgtgtct 1980
tcagccacag ccaccaccga acttggtagc gccgaggttg agagttcaca gaagcttctc 2040
ggcggagggt ttaaaagatt tacagagcta cttggatggc ttgggaaact aattggggga 2100
catgaggatg gtgacgaatg attgatttct ctgtttttgt tttgtctttt cttacaattt 2160
tatagtttgg ggtttggagg ttttgttctg ttaaattctc gcaatttgag taagttataa 2220
catgaggcgg catacaataa tttattgttt ggtaggatat atttttttgt cacttttgca 2280
taataataat tctgcttcag tct 2303

<210> 10
<211> 6714
<212> DNA
<213> Artificial
<220>
<223> Nucleotide sequence of the AtDBP1 gene (At2g25620) in the 8.1 mutant
(SALK_005240)

<400> 10
gccggcgaac agttttatta tctctctctt tctgcgtgtt ccaatttctt ccacaaaacc 60
ctaatttctt cttaaaccct gaaattcgtg taacctttca acaatctctt caattttgtt 120
ttcgattctg aatccattgt ttacgaatct ctcatctaaa gctacattct tcgttaaatc 180
gtttttaatc gactcgctca ctcgtgagtt gactcgtcag ctgtttccat aattgcttat 240
tgaattttta tacgatgatg tttcaatatg gaagaaacta gaggaatttc tgatccagag 300
aatgggagtt cgagttacgg cggtaaaccg ccgaatccac tctccttctc ttcttcctcc 360
gccgctgctg ccgtttacag gcaaaccttc gacggcgagc gatcgttggc gccgtgtaat 420
aagaggtcac tggttcgaca ctcatctctc gtaagattcg tttctcttca acttgatttg 480
tggatcagtt aggtttattt tgtgtattga atgatcaatt taagttgagg ttttgtaatt 540
attgctctgc ttttggattt gatcattatt ggtttcctaa tgtgtataat taaggtagaa 600
agtttctgtt tttaatgtgt cattatgaga tgtttgaggt tttctgattg attttcattt 660
tggtaggtga agacaatggt gtcagatata tctgttgaga atgagtttac tatagagaag 720
aacaagtctg agtttgttcc tgctcctgtg gttggcatgc acatacaaat ggacgaacgg 780
ataaaccttt tcacgccctt ttaaatatcc gattattcta ataaacgctc ttttctctta 840
ggtttacccg ccaatatatc ctgtcaaaca ctgatagttt aaactgaagg cgggaaacga 900
caatctgatc atgagcggag aattaaggga gtcacgttat gacccccgcc gatgacgcgg 960
gacaagccgt tttacgtttg gaactgacag aaccgcaacg ttgaaggagc cactcagccg 1020
cgggtttctg gagtttaatg agctaagcac atacgtcaga aaccattatt gcgcgttcaa 1080
aagtcgccta aggtcactat cagctagcaa atatttcttg tcaaaaatgc tccactgacg 1140
ttccataaat tcccctcggt atccaattag agtctcatat tcactctcaa tccaaataat 1200
ctgcaccgga tctggatcgt ttcgcatgat tgaacaagat ggattgcacg caggttctcc 1260
ggccgcttgg gtggagaggc tattcggcta tgactgggca caacagacaa tcggctgctc 1320
tgatgccgcc gtgttccggc tgtcagcgca ggggcgcccg gttctttttg tcaagaccga 1380
cctgtccggt gccctgaatg aactgcagga cgaggcagcg cggctatcgt ggctggccac 1440
gacgggcgtt ccttgcgcag ctgtgctcga cgttgtcact gaagcgggaa gggactggct 1500
gctattgggc gaagtgccgg ggcaggatct cctgtcatct caccttgctc ctgccgagaa 1560
agtatccatc atggctgatg caatgcggcg gctgcatacg cttgatccgg ctacctgccc 1620
attcgaccac caagcgaaac atcgcatcga gcgagcacgt actcggatgg aagccggtct 1680
tgtcgatcag gatgatctgg acgaagagca tcaggggctc gcgccagccg aactgttcgc 1740
caggctcaag gcgcgcatgc ccgacggcga tgatctcgtc gtgacccatg gcgatgcctg 1800
cttgccgaat atcatggtgg aaaatggccg cttttctgga ttcatcgact gtggccggct 1860
gggtgtggcg gaccgctatc aggacatagc gttggctacc cgtgatattg ctgaagagct 1920
tggcggcgaa tgggctgacc gcttcctcgt gctttacggt atcgccgctc ccgattcgca 1980
gcgcatcgcc ttctatcgcc ttcttgacga gttcttctga gcgggactct ggggttcgaa 2040
atgaccgacc aagcgacgcc caacctgcca tcacgagatt tcgattccac cgccgccttc 2100
tatgaaaggt tgggcttcgg aatcgttttc cgggacgccg gctggatgat cctccagcgc 2160
ggggatctca tgctggagtt cttcgcccac gggatctctg cggaacaggc ggtcgaaggt 2220
gccgatatca ttacgacagc aacggccgac aagcacaacg ccacgatcct gagcgacaat 2280
atgatcgggc ccggcgtcca catcaacggc gtcggcggcg actgcccagg caagaccgag 2340
atgcaccgcg atatcttgct gcgttcggat attttcgtgg agttcccgcc acagacccgg 2400
atgatccccg atcgttcaaa catttggcaa taaagtttct taagattgaa tcctgttgcc 2460
ggtcttgcga tgattatcat ataatttctg ttgaattacg ttaagcatgt aataattaac 2520
atgtaatgca tgacgttatt tatgagatgg gtttttatga ttagagtccc gcaattatac 2580
atttaatacg cgatagaaaa caaaatatag cgcgcaaact aggataaatt atcgcgcgcg 2640
gtgtcatcta tgttactaga tcgggcctcc tgtcaatgct ggcggcggct ctggtggtgg 2700
ttctggtggc ggctctgagg gtggtggctc tgagggtggc ggttctgagg gtggcggctc 2760
tgagggaggc ggttccggtg gtggctctgg ttccggtgat tttgattatg aaaagatggc 2820
aaacgctaat aagggggcta tgaccgaaaa tgccgatgaa aacgcgctac agtctgacgc 2880
taaaggcaaa cttgattctg tcgctactga ttacggtgct gctatcgatg gtttcattgg 2940
tgacgtttcc ggccttgcta atggtaatgg tgctactggt gattttgctg gctctaattc 3000
ccaaatggct caagtcggtg acggtgataa ttcaccttta atgaataatt tccgtcaata 3060
tttaccttcc ctccctcaat cggttgaatg tcgccctttt gtctttggcc caatacgcaa 3120
accgcctctc cccgcgcgtt ggccgattca ttaatgcagc tggcacgaca ggtttcccga 3180
ctggaaagcg ggcagtgagc gcaacgcaat taatgtgagt tagctcactc attaggcacc 3240
ccaggcttta cactttatgc ttccggctcg tatgttgtgt ggaattgtga gcggataaca 3300
atttcacaca ggaaacagct atgaccatga ttacgccaag cttgcatgcc tgcaggtccc 3360
cagattagcc ttttcaattt cagaaagaat gctaacccac agatggttag agaggcttac 3420
gcagcaggtc tcatcaagac gatctacccg agcaataatc tccaggaaat caaatacctt 3480
cccaagaagg ttaaagatgc agtcaaaaga ttcaggacta actgcatcaa gaacacagag 3540
aaagatatat ttctcaagat cagaagtact attccagtat ggacgattca aggcttgctt 3600
cacaaaccaa ggcaagtaat agagattgga gtctctaaaa aggtagttcc cactgaatca 3660
aaggccatgg agtcaaagat tcaaatagag gacctaacag aactcgccgt aaagactggc 3720
gaacagttca tacagagtct cttacgactc aatgacaaga agaaaatctt cgtcaacatg 3780
gtggagcacg acacacttgt ctactccaaa aatatcaaag atacagtctc agaagaccaa 3840
agggcaattg agacttttca acaaagggta atatccggaa acctcctcgg attccattgc 3900
ccagctatct gtcactttat tgtgaagata gtggaaaagg aaggtggctc ctacaaatgc 3960
catcattgcg ataaaggaaa ggccatcgtt gaagatgcct ctgccgacag tggtcccaaa 4020
gatggacccc cacccacgag gagcatcgtg gaaaaagaag acgttccaac cacgtcttca 4080

aagcaagtgg attgatgtga tatctccact gacgtaaggg atgacgcaca atcccactat 4140
ccttcgcaag acccttcctc tatataagga agttcatttc atttggagag aacacggggg 4200
actctagagg atccccgggt accgagctcg aatttccccg atcgttcaaa catttggcaa 4260
taaagtttct taagattgaa tcctgttgcc ggtcttgcga tgattatcat ataatttctg 4320
ttgaattacg ttaagcatgt aataattaac atgtaatgca tgacgttatt tatgagatgg 4380
gtttttatga ttagagtccc gcaattatac atttaatacg cgatagaaaa caaaatatag 4440
cgcgcaaact aggataaatt atcgcgcgcg gtgtcatcta tgttactaga tcgggaattc 4500
actggccgtc gttttacaac gtcgtgactg ggaaaaccct ggcgttaccc aacttaatcg 4560
ccttgcagca catccccctt tcgccagctg gcgtaatagc gaagaggccc gcaccgatcg 4620
cccttcccaa cagttgcgca gcctgaatgg cgcccgctcc tttcgctttc ttcccttcct 4680
ttctcgccac gttcgccggc tttccccgtc aagctctaaa tcgggggctc cctttagggt 4740
tccgatttag tgctttacgg cacctcgacc ccaaaaaact tgatttgggt gatggttcac 4800
gtagtgggcc atcgccctga tagacggttt ttcgcccttt gacgttggag tccacgttct 4860
ttaatagtgg actcttgttc caaactggaa caacactcaa ccctatctcg ggctattctt 4920
ttgatttata agggattttg ccgatttcgg aaccaccatc aaacaggatt ttcgcctgct 4980
ggggcaaacc agcgtggacc gcttgctgca actctctcag ggccaggcgg tgaagggcaa 5040
tcagctgttg cccgtctcac tggtgaaaag aaaaaccacc ccagtacatt aaaaacgtcc 5100
gcaatgtgtt attaagttgt ctaagcgtca atttgtttac accacaatat atcctacgcg 5160
ttctggagct tggtctgata ttggctccag gtcaagcatg gaagatgctt atctatgcgt 5220
tgataatttc atggatagct ttggccttct gaattctgag gctggtccaa gtgccttcta 5280
tggggtatgt tctttcttca gttttcctga ttttttgtag atttgtagca taagcaaaca 5340
actgtgaatg tagtgaaaat atggggatct cttattgaat tttgtttgtt attaggtatt 5400
tgatggacat ggtgggaagc atgccgctga gtttgcatgt caccatatac cgaggtacat 5460
tgttgaagat caagagtttc ctagtgaaat caataaggtg ttatcttcag catttcttca 5520
aacagacact gccttcttag aggcgtgttc attggatggg agccttgctt caggaactac 5580
tgctttggca gctattcttt ttggaaggtt tgttgattca actcttatta tcttttagtt 5640
ttgtctaacc taaactctta catcttggtt atgttaaaga aaaacgtgtt tgggaataga 5700
tgcttatcct caactgcgta gtgtgcataa tcttgacctt taacacaatc atgtacaact 5760
aattcgcctg tagatgtatt ctctgcccat atgtgctgct gctataatgt agttattgtc 5820
tcatatggtc tttgagtcac gtatatactt gctcatttca tactctatta gtattctggt 5880
atgaaaccgt ttggtaccgt gttaacatat agttttgtgg taacaggtcg ttggtggtag 5940
caaacgctgg agattgcaga gcagtcttat cccgtcaggg aaaagccatt gaaatgtcaa 6000
gagaccacaa acccatgagc agtaaggaaa ggagacgcat tgaagcatcg ggtggacatg 6060
tattcgatgg ctatctaaat ggacaactta atgtggctcg agcgctaggt gactttcata 6120
tggaaggcat gaagaagaag aaagatggtt ctgattgtgg acctctaatt gcagagcctg 6180
agctcatgac aacaaaacta acagaagagg atgagttcct tataattggg tgtgacgggg 6240
tttgggatgt gttcatgagc cagaatgctg tagattttgc cagaaggaga ctacaggagc 6300
acaatgaccc ggtcatgtgt agtaaggagc tggttgagga agctttgaag aggaagagtg 6360
ctgataatgt gacggcagtg gttgtgtgtc ttcagccaca gccaccaccg aacttggtag 6420
cgccgaggtt gagagttcac agaagcttct cggcggaggg tttaaaagat ttacagagct 6480
acttggatgg cttgggaaac taattggggg acatgaggat ggtgacgaat gattgatttc 6540
tctgtttttg ttttgtcttt tcttacaatt ttatagtttg gggtttggag gttttgttct 6600
gttaaattct cgcaatttga gtaagttata acatgaggcg gcatacaata atttattgtt 6660
tggtaggata tatttttttg tcacttttgc ataataataa ttctgcttca gtct 6714

## Description

### TECHNICAL FIELD OF THE INVENTION

This invention relates to the use of a mutation in order to generate virus-resistant mutant plants, specifically, the use of the 8.1 mutation of the AtDBP1 gene of *Arabidopsis thaliana* as a regulator of plant resistance to potyviruses in order to modify the plant phenotype, as well as to the genetically-modified plants obtained, which exhibit a higher resistance to potyvirus infection than unmodified plants.

### BACKGROUND OF THE INVENTION

In nature, all living organisms must constantly defend against their interaction with others in order to survive. Plants co-exist with numerous pathogenic microorganisms, such as bacteria, viruses, fungi, etc.; however, in the vegetable world, disease is an exception, due to plants' numerous defence mechanisms against these pathogens. Some of these mechanisms are constitutive, being established in the plant prior to the arrival of the pathogen, whereas others are induced following the perception thereof and confer protection not only at the infection site, but systemically throughout the entire plant (Dempsey et al., Crit. Rev. Plan Sci. Vol. 18, pp. 547-575, 1999). Knowledge about the signalling pathways involved in the defensive response and the processes that underlie the interaction between the plant and the pathogen has unquestionable interest, both basic and applied. This would facilitate the design of strategies aimed at obtaining vegetable varieties with a higher resistance against pathogens, as well as the control of diseases in croppable species in order to increase the yield, the quality and the safety of the crops, since it is estimated that yield losses caused by pathogens represent about 10%-20% (Kreps et al., Plant Physiol. Vol. 130, pp. 2129-2141, 2002).

The diseases produced by viruses in plants may damage the leaves, the stems, the roots, the fruits, the seeds or the flowers, and are responsible for a significant percentage of the economic losses caused by a reduction in the yield of crops and in the quality thereof (Agrios, 1988. Plant Pathology, 3rd Ed., San Diego, CA, Academic Press, p. 655). For this reason, plant viruses are considered to be the greatest threat for modern agriculture, since they lead to reductions in productivity and imperfect or low-quality products in many crops and ornamental plants throughout the world. Given the generalised, persistent character of the diseases caused by these pathogens, it is necessary to establish adequate control methods designed to prevent infections, protect the crops and thereby minimise agricultural losses.

Plant viruses are classified into RNA viruses and DNA viruses, on the basis of the nature of the genetic material thereof, the most frequent being single-stranded RNA viruses of positive polarity (Hull, 2002. Matthews' Plant Virology, Academic Press). Infection is produced through wounds caused by physical damages due to the environment or the action of vectors such as insects, mites, nematodes and certain fungi that live on the soil. In the cytoplasm, the RNA virus is disassembled, replicates, translates its messengers into proteins and is locally and systemically mobilised (Stange, Cien. Inv. Agr. Vol. 33, pp. 1-18, 2006). In order to reproduce, the virus uses energy and proteins from the host cell. During each stage of the viral cycle, complex interactions are generated between the host plant and the virus, which have been the subject of intense study in recent years. For example, certain components of the host plant, such as microtubules, actin/myosin filaments and calreticulin, facilitate infection and the movement of the virus in the plant (Boevnik and Oparka, Plant Physiol. Vol. 138, pp. 1815-1821, 2005; Chen et al., Plant Physiol. Vol. 138, pp. 1866-1876, 2005). However, the identity of all the factors inherent to the host that are involved in the viral cycle is still unknown. Without a doubt, this knowledge is one of the greatest challenges in the field of virology in order to generate resistance to viral infections in crops of economic and environmental interest.

In order to overcome the slowness and complexity of classic genetic improvement programmes, biotechnological strategies have been developed based on obtaining transgenic plants in order to generate resistance to viral infections. In this regard, the concept that has been most successfully used is the concept of virus-derived resistance proposed by Sanford and Johnston (J. Theor. Biol. Vol. 115, pp. 395-405, 1985), which is based on the expression of viral genes in plants. This could interfere with the viral cycle or activate gene-silencing mechanisms. Post-transcriptional gene silencing (PTGS), also known as RNA silencing, is an essential antiviral defence mechanism in plants. It is a process that controls gene expression at the post-transcriptional level and leads to the suppression of foreign genetic elements, such as viruses and transposons, through a specific RNA degradation mechanism (Baulcombe, Nature. Vol. 431, pp. 356-363, 2004; and Trends Biochem. Sci. Vol. 30, pp. 290-293, 2005).

In vegetable organisms, at least three RNA silencing pathways are known: cytoplasmic silencing by small interfering RNAs (siRNAs), silencing of endogenous mRNAs by microRNAs (miRNAs) and silencing associated with the methylation of DNA and the suppression of transcription. All these pathways involve the breakdown of double-stranded RNA molecules (dsRNAs) into small RNAs, such as siRNAs and miRNAs (Baulcombe, Nature. Vol. 431, pp. 356-363, 2004). The limitations presented by technologies based on RNA silencing include the requirement of a high sequence specificity for RNA degradation (Ritzenthaler, Curr. Opin. Biotechnol. Vol. 16, pp. 118-122, 2005). Moreover, viruses have developed the capacity to encode suppressors of this innate antiviral defence mechanism. Amongst suppressor proteins, the HCPro (helper component protease) protein encoded by potyviruses (and Vaucheret, Science. Vol. 292, pp. 2277-2280, 2001) has been one of the most widely studied. For all these reasons, the application of these strategies could be limited and not very effective to generate resistance against a wide range of viruses.

In other cases, the expression of plant resistance genes, or R genes, has been used. These genes are responsible for the recognition of the invader by means of direct or indirect interaction of the product of the expression thereof with avirulence factors (Avr) of the pathogen. Said recognition triggers a cell death programme at the infection site or a hypersensitive reaction which blocks dispersion of the pathogen and leads to the activation of the defensive response. Resistance genes to certain viruses have been identified in different vegetable species, such as the tobacco N gene (*Nicotiana tabacum*), which confers resistance to the tobacco mosaic virus (TMV), being a classic model in the study of plant-virus interactions (Whitham et al., Cell. Vol. 78, pp. 1101-1115, 1994; and Proc. Natl. Acad. Sci. USA. Vol. 93, pp. 8776-8781, 1996; Dineshkumar et al., Proc. Natl. Acad. Sci. USA. Vol 92, pp. 4175-4180, 1995). Genes with structural similarity, called resistance gene analogues (RGA), have also been identified in species of agricultural interest (Shen et al., Mol. Plant Microbe Interact. Vol. 11, pp. 815-823, 1998; Deng et al., Theor. Appl. Genet. Vol. 101, pp. 814-822, 2000; Di Gaspero and Cipriani, Theor. Appl. Genet. Vol. 106, pp. 163-172, 2002; Baldi et al., Theor. Appl. Genet. Vol. 109, pp. 231-239, 2004; Dondini et al., J. Horticul. Science and Biotech. Vol. 79, pp. 729-734, 2004; Soriano et al., Theor. Appl. Genet. Vol. 110, pp. 980-989, 2005). However, since, in general, resistance genes specifically recognise certain pathogen strains, their general applicability is very limited. On the other hand, the rapid evolution of viral strains capable of avoiding the plant's resistance mechanisms requires new concepts in the development of pathogen-resistant vegetable varieties.

By contrast with the dominant resistance mediated by R genes and RNA silencing, there are alternative resistance mechanisms that, due to their special characteristics, are called recessive-nature mechanisms. Recessive resistance would be the result of a passive mechanism that makes a plant resistant due to the lack of a host-specific factor required by the virus to complete its cycle, or due to the presence of a mutated version of this factor (Fraser, Annu. Rev. Phytopathol. Vol. 28, pp. 179-20, 1990; and *Academic Press,* pp. 1300-1307. San Diego, CA, 1999). Potyviruses represent approximately 30% of all the known plant viruses and, as a group, are very destructive in agriculture (Ward *et al.,* 1991).

There is a less information available, and it is more disperse, about incompatible interactions between viruses and plants controlled by recessive resistance genes in the host (Díaz-Pendón et al., 2004. Mol. Plant Pathol. Vol. 5, pp. 223-233, 2004). Viruses are dependent on the host's biochemical machinery to complete their biological cycle. Thus, successful infection of a plant requires a number of compatible interactions between host factors and viral factors throughout a complex process that includes the expression and replication of the viral genome, cell-to-cell movement and long-distance translocation through the plant's vascular system (Carrington et al., Plant Cell. Vol. 8, pp. 1669-1681, 1996; Maule et al., Curr. Opin. Plant Biol. Vol. 5, pp. 279-284, 2002).

Some of these host factors required for gene expression or replication of the virus have been identified by analysing mutagenised populations. Thus, it has been demonstrated that, in *Arabidopsis thaliana,* TOM1 and TOM2A are required for an efficient multiplication of the tobacco mosaic virus in *Arabidopsis* protoplasts. These are host transmembrane proteins that interact with one another and with viral replication proteins encoded by the virus, being essential to form the tobamovirus replication complex (Hagiwara et al., EMBO J. Vol 22, pp. 344-353, 2003; Ishikawa et al., J. Virol. Vol. 67, pp. 5328-5338, 1991; Mol. Gen. Genet. Vol. 230, pp. 33-38, 1993; Ohshima et al., Virology Vol. 243, pp. 472-481, 1998; Tsujimoto et al., EMBO J. Vol. 22, pp. 335-343, 2003; Yamanaka et al., Proc. Natl. Acad. Sci. USA. Vol. 97, pp. 10107-10112, 2000; and J. Virol. Vol. 76, pp. 2491-2497, 2002). With a similar approach, it has also been demonstrated that one of the isoforms of eukaryotic translation initiation factor eIF(iso)4E is necessary for the multiplication of the *Turnip mosaic potyvirus* (TuMV) and the *Tobacco etch virus* (TEV) (Duprat et al., Plant J. Vol. 32, pp. 927-934, 2002; Lellis et al., Curr. Biol. Vol. 12, pp. 1046-1051, 2002; Whitham et al., Proc. Natl. Acad. Sci. USA. Vol. 96, pp. 772-777, 1999). Moreover, several *Arabidopsis* mutants have been identified wherein viral movement is restricted, such as *cum1-1* and *cum2-1,* where the propagation of the *Cucumber mosaic virus* (CMV) (Yoshii et al., Plant J. Vol. 13, pp. 211-219, 1998a), and that of CMV and the *Turnip crinkle virus* (TCV) are affected (Yoshii et al., J. Virol. Vol. 72, pp. 8731-8737, 1998b), respectively; and *vsm1,* wherein the systemic movement of a tobamovirus is specifically affected (Larley et al., Mol. Plant-Microbe Interact. Vol. 11, pp. 706-709, 1998). The *cum1-1* and *cum2-1* mutants have the eIF4E and eIF4G genes altered, respectively (Yoshii et al., J. Virol. Vol. 78, pp. 6102, 2004). Most cases of recessive resistance have been described in cultivated species, but genes involved therein have only been identified in peppers and lettuce, in both cases being translation initiation factor eIF4E, and against potyvirus (Nicaise et al., Plant Physiol. Vol. 132, pp. 1272-1282, 2003; Ruffel et al., Plant J. Vol. 32, pp. 1067-1075, 2002). Therefore, although there is scant information available about the mechanisms responsible for this, there seems to be a close link between translation initiation factors and resistance to viruses (Robaglia and Caranta, Trends Plant Sci. Vol. 11, pp. 40-45, 2006).

Patent application WO 01/34823 A2, titled: "Method for enhancing resistance in plants", relates to the induction in plants of resistance to a wide range of pathogens, specifically, viruses, bacteria and fungi, by means of the viral expression of a polyprotein encoded by members of the potyvirus group. Said method for the protection of plants against pathogens comprises: a) transforming a plant cell with a functional gene or other gene silencer suppressors, b) identifying the plant's transformed cells, and c) regenerating a transformed plant from the transformed cell.

Document US 5,986,175, titled "Virus resistant plants", describes and claims a method of conferring resistance to plants by means of the expression in the plant of an isolated DNA sequence that comprises nucleotides which encode a potyvirus replicase, and cites a large number of potyviruses. The same authors, in patent document US 5,589,612, confer potyvirus resistance to plants using a potyvirus protease in the method.

Document WO 95/04825 A1, titled "Improvements in or relating to disease resistance of plants", also describes the increased resistance conferred to plants by means of a potyvirus protease. Specifically, it describes a DNA molecule that encodes a portion of potyvirus replicase which, once introduced into the appropriate plant, is capable of increasing said plant's resistance to viral diseases. It also describes transgenic plants that have increased resistance to viral diseases.

Patent application WO 94/16097 A1, titled "Plantes transgéniques résistantes aux virus végétaux et procédé d'obtention", describes and claims a potyvirus-resistant plant that contains, in its genome, one or more DNA fragments that express the transcripts corresponding to a protein or part of a protein from a donour virus, except for a capsid protein and the entire Nla protease of TMV (Tobacco Mosaic Virus), said plant being obtained by the introduction of vectors obtained from the donour virus.

Granted patent document ES 2 166 361 T3, titled "Producción de plantas resistentes a los virus a través de la introducción de RNA viral intraducible de sentido positivo", describes and claims a method of producing a plant with reduced susceptibility to viral infection, which comprises: a) transforming plant cells with a DNA molecule that encodes untranslatable positive-sense viral RNA molecules, wherein said RNA molecule is derived from the nucleotide sequence of a plant virus gene, and b) regenerating a plant that comprises the transformed plant cell. The untranslatable positive-sense viral RNA molecule may be derived from a potyvirus; and it may also contain at least one mutation that makes the RNA molecule untranslatable, and the expression of said RNA molecule inside the plant reduces the plant's susceptibility to viral infection.

Patent application WO 2004/057941 A2, titled: "Recessive plant viral resistance results from mutations in translation initiation factor eIF4E", relates to a method of conferring virus resistance to plants, primarily potyvirus resistance. The method involves the silencing of the gene that encodes the plant's transcription initiation factor eIF4E, under effective conditions to confer virus resistance to the plant.

This silencing comprises: supplying a transgenic plant or plant seed transformed with heterologous nucleic acid molecules which silence the gene that encodes eIF4E in the plant and, subsequently, growing the transgenic plant or the transgenic plant grown from the transformed seed under effective conditions to confer virus resistance to said transgenic plant. Said heterologous molecules may be anti-sense or sense oligonucleotides that are complementary (different degrees of complementarity being admitted in the sequence) to the mRNA that encodes factor eIF4E. Said nucleic acid may also be iRNA (interference RNA). Both the transgenic plant and the plant seed transformed with the heterologous molecules may be selected from a broad group that includes *Arabidopsis thaliana.*

Another aspect of this method comprises the overexpression of a nucleic acid molecule that encodes a heterologous transcription initiation factor, eIF4E, as well as an expression system that contains the gene construct and the host cell transformed with the gene construct.

The document describes and claims the gene constructs, the expression system that comprises the gene constructs, the transformed host cell, the transformed plants, etc., which participate in the achievement of potyvirus resistance.

In sum, the document describes and claims the obtainment of recessive potyvirus resistance in plants thanks to mutations in translation initiation factor eIF4E.

Document WO 03/000898 A1, titled: "Plant genes involved in defense against pathogens", presents the use of different genes to confer resistance to certain types of potyviruses (amongst other pathogens) by means of the repression thereof (amongst other possibilities).

The invention describes 228 genes identified as useful to confer resistance to more than one pathogen (bacteria, *oomycetes* and viruses). It also identifies genes that are overexpressed or the expression whereof is reduced in response to viral infections.

Therefore, the invention provides both transgenic plants the genome whereof is increased by a nucleic acid molecule of the invention and transgenic plants the corresponding gene whereof is interrupted in the genome, leading to the loss, reduction or alteration of the function of the product encoded by said gene (as compared to the wild plant), these plants acquiring resistance or tolerance capacity toward certain viruses or other pathogens.

The pathogens may be bacteria, fungi or viruses. Amongst the possible viruses, different types of potyviruses are cited.

The document presents the possibility to repress *Arabidopsis* genes as well as genes that encode DNA-binding proteins and phosphatases. However, none of the genes presented correspond to AtDBPI.

In the article "A novel transcription factor involved in plant defense endowed with protein phosphatase activity" (Carrasco et al., The EMBO Journal. Vol. 22, No. 13, pp. 3376-3384, 2003), the same authors of this application describe the DBP1 protein of tobacco plants for the first time.

This article focuses on the repressive effect of transcription factor DBP1 on the transcription of the CEVI1 gene (which encodes a protein that is expressed in the response toward compatible plant-virus interactions). In said document, by means of *in vitro* studies, they have shown that DBP1 specifically binds to sequences of the CEVI1 gene promoter.

Moreover, in order to demonstrate the relationship between DBP1 and the transcriptional regulation of CEVI1, transgenic tobacco plants are constructed which present an anti-sense DBP1 construct wherein the expression level of the DBP1 gene is severely reduced. In these plants, it is shown that, in the absence of DBP1, there is an accumulation of CEVI1 (which suggests the repressive role of this new regulator in the transcriptional control of target genes).

In the work developed, they refer to PP2Cs as negative modulators of stress signalling pathways in plants (amongst others). Furthermore, they show CEVI1 to be a defence-related gene, which may be induced as a consequence of viral infection and the transcription whereof may be regulated by DBP1.

Special attention is given to CEVI1 and its role in controlling susceptibility to diseases in plants, but they do not mention the possibility of using the repression of the expression of DBP1 as a mechanism to confer potyvirus resistance to plants, nor a potential relationship between DBP1 and eIF(iso)4E.

This article makes no reference to AtDBP1 (that is, to DBP1 from *Arabidopsis thaliana*) nor possible isoforms of DBP1.

In the article "A novel DNA-Binding Motif, Hallmark of a New Family of Plant Transcription Factors" (Carrasco et al., Plant Physiology. Vol. 137, pp. 602-606, 2005), the authors of this application describe a new family of plant-specific transcription factors, the DBP₁ family of transcription factors, which includes AtDBP1. They indicate the conservation of the DNA-binding domain of these transcription factors as the basis for future studies of the regulatory functions of said factors. During the study, they create two mutated versions of DBP1 from tobacco: mut1 and mut2, wherein they substitute certain amino acids from the conserved N-terminal region by alanine, giving rise to proteins that lose their DNA-binding capacity.

The article shows that 5 genes have been identified in *Arabidopsis* which encode proteins related to DBP1. Amongst them, that which shows the greatest similarity to DBP1 is AtDBP1 (At2g25620; GenBank, accession number: NM_128120[GenBank]).

Finally, in the article "14-3-3 Mediates Transcriptional Regulation by Modulating Nucleocytoplasmic Shuttling of Tobacco DNA-binding Protein Phosphatase-1" (Carrasco et al., J. Biol. Chem. Vol. 281, Issue 32, 22875-22881, 2006), the authors of this patent application identify the G isoform of the tobacco 14-3-3 protein as the first protein that interacts with the DBP factor. They use said protein to understand the mechanism that underlies this family of transcriptional regulators.

This article presents the interaction of both DBP1 and AtDBP1 with their corresponding 14-3-3 isoforms, an interaction that positively regulates the induction of the CEVI1 gene. In order to locate the binding area between DBP1 and 14-3-3, they construct deleted mutants in DBP1: 1 (which maintains the N-terminus and therefore interacts with 14-3-3) and 2 (deleted at the N-terminus and which does not bind to 14-3-3). In order to determine the exact position, several deletions are made.

Thus, they perform tests with 14-3-3 λ from *Arabidopsis* (functional ortologue of 14-3-3G from tobacco). They compare the interaction of AtDBP1 with 14-3-3 λ and show that both proteins interact through the N-terminal region. These results reinforce the biological significance of the DBP1-14-3-3G interaction.

In sum, they conclude that the 14-3-3G isoform positively regulates the induction of the tobacco CEVI1 gene by direct interaction with DBP1.

### OBJECT OF THE INVENTION

The object of this invention relates to the use of the 8.1 mutation of the AtDBP1 gene of *Arabidopsis thaliana* (Gen At2g25620; Gen Bank, accession number NM_128120) to modify the plant phenotype in such a way that the resulting plant exhibits increased resistance to potyvirus infection as compared to the wild-type plant.

Another object of this invention relates to the use of the AtDBP1 gene in species of agricultural and industrial interest as a regulator of the response to potyviruses in plants. Such use entails the loss of function of the AtDBP1 gene, leading to a lower accumulation of eIF(iso)4E, which would lead to resistance or decreased susceptibility to potyviruses.

A regulator is understood to be the gene and, therefore, the protein encoded thereby, which regulates the mechanism or part of the mechanism that underlies and is used by the viruses in question in this study to penetrate into, multiply and infect the plant.

In the description of the invention, studies are performed of the involvement of the *AtDBP1* gene in the resistance or decreased susceptibility to potyviruses. The experiments are performed with the 8.1 mutant line of *Arabidopsis thaliana,* wherein the *AtDBP1* gene is interrupted by the insertion of T-DNA in the second exon of its encoding sequence, interrupting the N-terminal region of the protein at the DNC motif, such that the accumulation of its mRNA is inhibited. That is, a loss of function characterised by non-expression of mRNA. The mutant plants are exposed to two different types of potyviruses, and it is verified that in fact their resistance or lack of susceptibility to said potyviruses increases.

Another object of the invention is the use of the 8.1 mutation of the AtDBP1 gene of *Arabidopsis thaliana* to generate plants that present higher resistance to the Plum Pox Potyvirus (PPV) than the wild-type plant. Said resistance is detected by means of RT-PCR analysis.

Finally, another object of the invention is the use of the 8.1 mutation of the AtDBP1 gene of *Arabidopsis thaliana* to generate plants that exhibit attenuated symptoms following infection with the Turnip Mosaic Potyvirus (TuMV), which does not occur in the wild-type plant.

### DESCRIPTION OF THE FIGURES

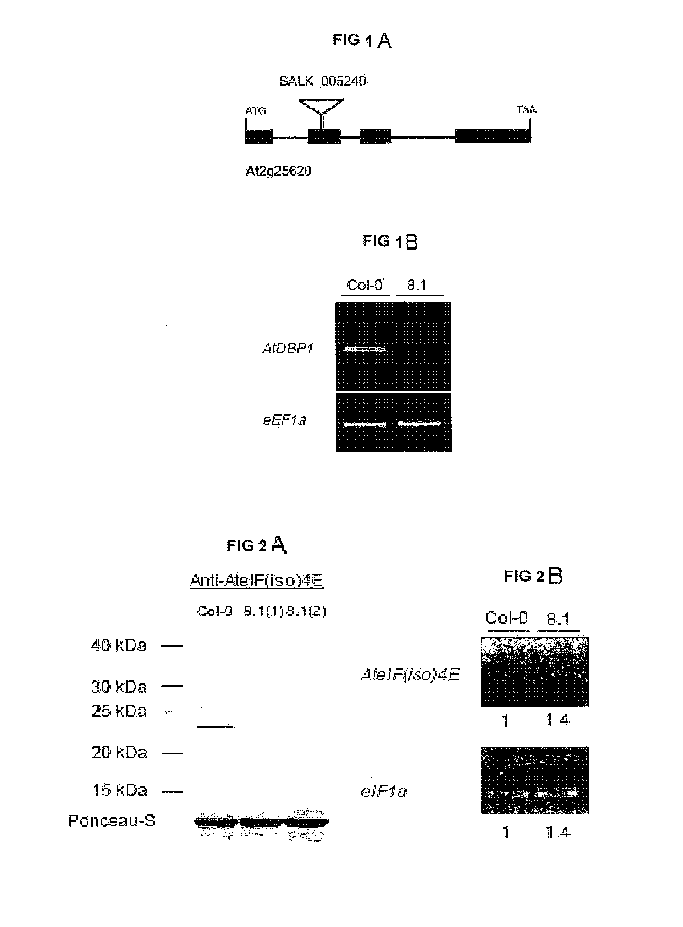

Figure 1. Characterisation of the 8.1 insertion mutant, which carries a T-DNA insertion in the *AtDBP1* gene. A) Structure of the *AtDBP1* gene. The *AtDBP1* gene has 4 exons (which are indicated with black rectangles) separated by 3 introns. The 8.1 line (SALK_005240) has a T-DNA insertion that interrupts the sequence that encodes the *AtDBP1* gene at the second exon. B) Analysis of the expression of the *AtDBP1* gene in the 8.1 insertion line. Agarose gel electrophoresis of the amplification products obtained by RT-PCR using specific primers for the *AtDBP1* gene as compared to Col-0 wild plants.
Figure 2. Analysis of the expression of AteIF(iso)4E in the *AtDBP1* 8.1 insertion line. A) Western-blot of protein extracts obtained from Col-0 wild plant leaves and two different batches of plants from the 8.1 mutant line, 8.1(1) and 8.1(2). On the left, the migration of the marker proteins used is shown, as a function of their molecular size. As a load control, a detail of the staining of the nitrocellulose membrane with Ponceau-S following the transfer is shown. B) Analysis of the accumulation of messenger RNA of AteIF(iso)4E in Col-0 wild plants and in the 8.1 insertion line by RT-PCR. Agarose gel electrophoresis of the amplification products obtained using specific primers for the *AteIF(iso)4E* gene (top) and the *AteEFla* gene, which is included as a control (bottom), as compared to Col-0 wild plants. Under the gel detail, the relative quantification of each amplification product is shown as compared to that obtained from Col-0 plants.

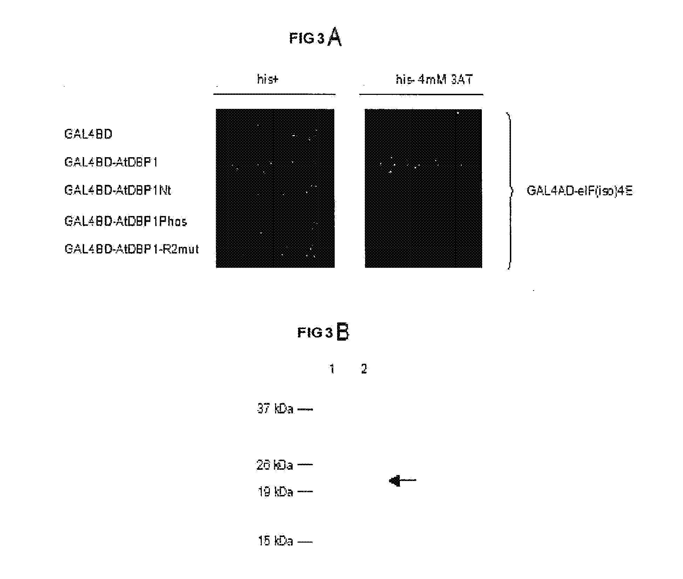

Figure 3. Analysis of the interaction between AtDBP1 and AteIF(iso)4E. A) Yeast two-hybrid system: growth in the presence (left) and absence (right) of histidine of yeast strains that express different regions of the AtDBP1 protein fused to the DNA-binding domain of GAL4 and a fusion of AteIF(iso)4E to the transcription activation domain of the same factor. The yeast cells' capacity to grow in a histidine-free medium is dependent on the interaction between the 2 fusion proteins and the subsequent activation of the transcription of the *HIS3* marker gene. The histidine-free medium was supplemented with 3-amino-triazole, an inhibitor of the HIS3 enzyme, in order to increase the selective pressure. B) Co-immunoprecipitation of AtDBP1 and AteIF(iso)4E: Western-blot of the fractions immunoprecipitated with antibodies against the hemagglutinin (HA) epitope from leaf protein extracts obtained from Col-0 plants (1) and transgenic plants that express AtDBP1 fused to said epitope (2). The Western-blot was developed using anti-AteIF(iso)4E antibodies.

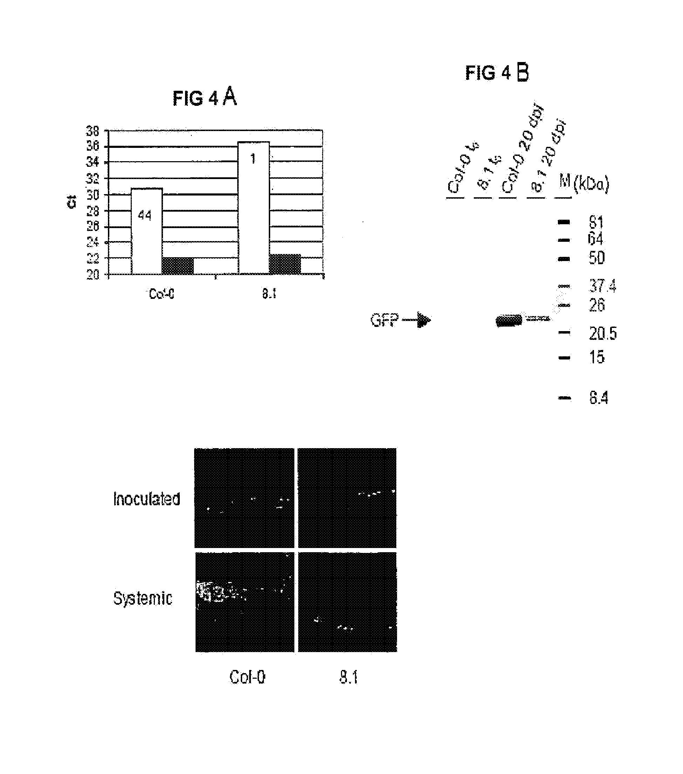

Figure 4. Analysis of the viral accumulation and distribution in 8.1 mutant line plants and Col-0 wild plants following inoculation with the sharka virus (Plum Pox Virus or PPV) fused to the GFP reporter. A) Accumulation of viral RNA: quantitative PCR amplification of cDNA obtained from non-inoculated leaves of Col-0 control plants and 8.1 insertion line plants infected with a version of the virus that includes the sequence which encodes the GFP green fluorescent protein (PPV-GFP), 20 days after inoculation. The white bars represent the amplification of the viral RNA using specific primers for GFP. As a control, the *ACTINA8* gene is included (black bars). The numbers over the bars indicate the quantification of the difference observed between Col-0 and 8.1 after normalising the results obtained with those of the respective control. B) Accumulation of viral protein: Western-blot of leaf protein extracts obtained before inoculating Col-0 and 8.1 plants with PPV-GFP (to) and 20 days after the inoculation (t₂₀), immunodecorated with anti-GFP polyclonal antibody. On the right, the migration of the molecular weight marker used is indicated. C) Analysis of the distribution of the virus in inoculated (upper panels) and non-inoculated or systemic (lower panels) leaves from Col-0 (left) and 8.1 (right) plants, by means of fluorescence microscopy for the detection of GFP.

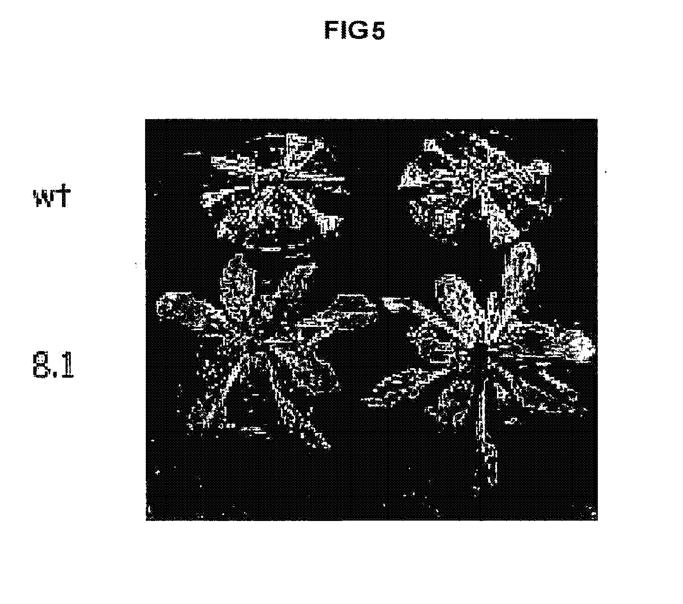

Figure 5. Infection with the Turnip Mosaic Virus (or TuMV). Symptoms observed in Col-0 (upper portion) and 8.1 (lower portion) plants following infection with TMV.

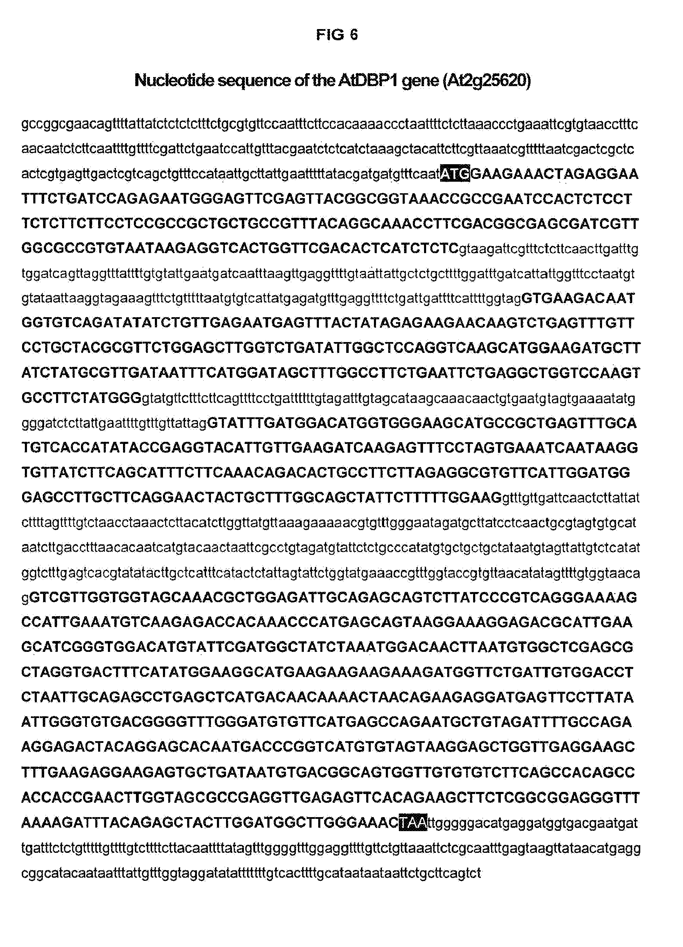

Figure 6. Nucleotide sequence of the AtDBP1 gene (At2g25620). The exons are indicated in bold, upper-case letters, and the introns and the untranslated 5'- and 3'-regions are indicated in lower-case letters.

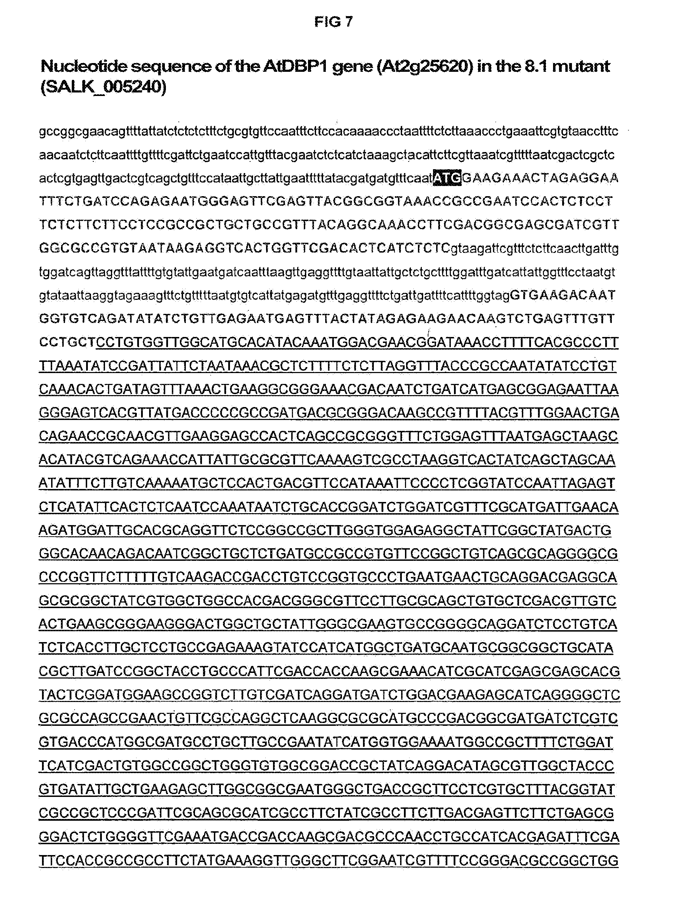

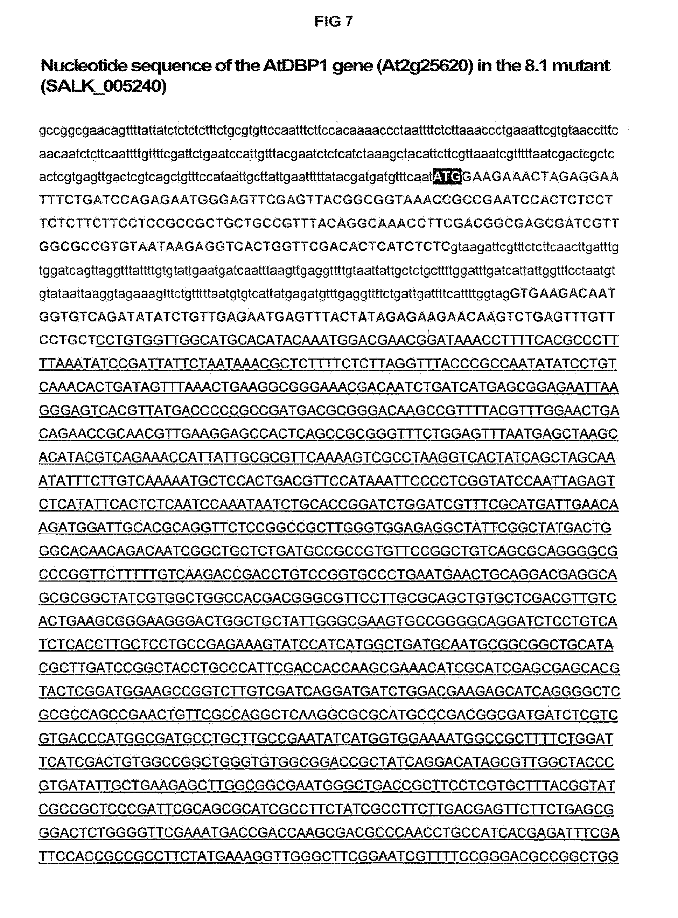

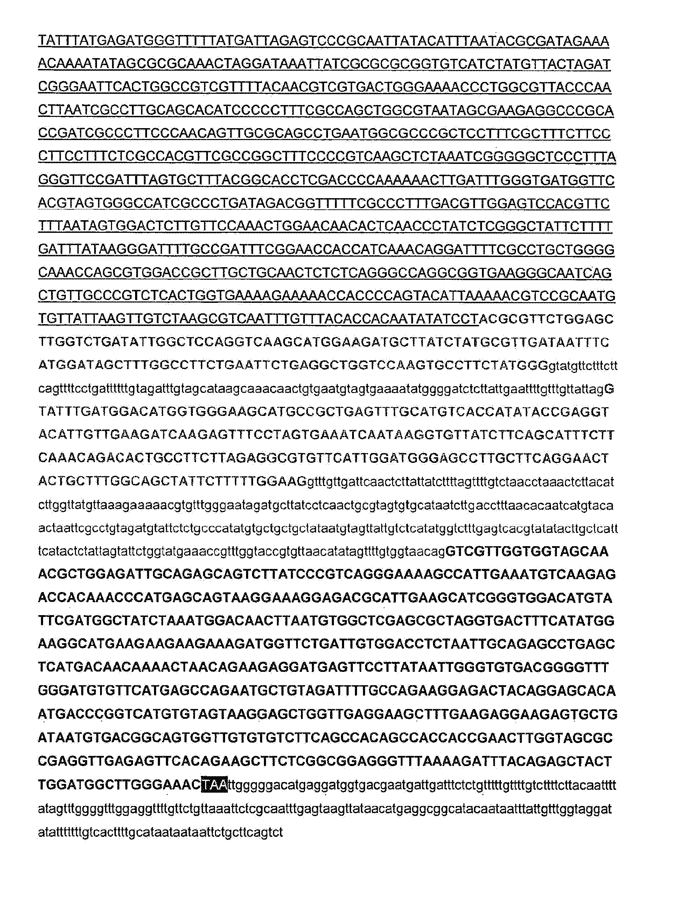

Figures 7. Nucleotide sequence of the AtDBP1 gene (At2g25620) in the 8.1 mutant (SALK_005240). The exons are indicated in bold, upper-case letters, the introns and the untranslated 5'- and 3'-regions are indicated in lower-case letters, and the sequence that would correspond to the T-DNA is underlined.

### DETAILED DESCRIPTION OF THE INVENTION

The potyvirus family represents a large number of plant viral pathogens that collectively may infect most cultivated species. Infection by potyvirus may induce a variety of symptoms, including foliar mottling, the distortion of seeds and fruits, and may thus severely compromise the yield and/or quality of the crop. The potyvirus family, or *Potyviridae,* includes the following definitive species: *Alstroemeria mosaic potyvirus, Amaranthus leaf mottle potyvirus, Araujia mosaic potyvirus, Arracacha Y potyvirus, Artichoke latent potyvirus, Asparagus 1 potyvirus, Banana bract mosaic potyvirus, Bean common mosaic necrosis potyvirus, Bean common mosaic potyvirus, Bean yellow mosaic potyvirus, Beet mosaic potyvirus, Bidens mosaic potyvirus, Bidens mottle potyvirus, Cardamom mosaic potyvirus, Carnation vein mottle potyvirus, Carrot thin leaf potyvirus, Cassava brown streak potyvirus, Cassia yellow spot potyvirus, Celery mosaic potyvirus, Chickpea bushy dwarf potyvirus, Chickpea distortion mosaic potyvirus, Clover yellow vein potyvirus, Commelina diffusa potyvirus, Commelina mosaic potyvirus, Cowpea green vein-banding potyvirus, Cowpea Moroccan aphid-borne mosaic potyvirus, Cowpea rugose mosaic potyvirus, Crinum mosaic potyvirus, Daphne Y potyvirus, Dasheen mosaic potyvirus, Datura Colombian potyvirus, Datura distortion mosaic potyvirus, Datura necrosis potyvirus, Datura shoestring potyvirus, Dendrobium mosaic potyvirus, Desmodium mosaic potyvirus, Dioscorea alata potyvirus, Dioscorea green banding mosaic potyvirus, Eggplant green mosaic potyvirus, Euphorbia ringspot potyvirus, Freesia mosaic potyvirus, Groundnut eyespot potyvirus, Guar symptomless potyvirus, Guinea grass mosaic potyvirus, Helenium Y potyvirus, Henbane mosaic potyvirus, Hippeastrum mosaic potyvirus, Hyacinth mosaic potyvirus, Iris fulva mosaic potyvirus, Iris mild mosaic potyvirus, Iris severe mosaic potyvirus, Johnsongrass mosaic potyvirus, Kennedya Y potyvirus, Leek yellow stripe potyvirus, Lettuce mosaic potyvirus, Lily mottle potyvirus, Maize dwarf mosaic potyvirus, Malva vein clearing potyvirus, Marigold mottle potyvirus, Narcissus yellow stripe potyvirus, Nerine potyvirus, Onion yellow dwarf potyvirus, Ornithogalum mosaic potyvirus, Papaya ringspot potyvirus, Parsnip mosaic potyvirus, Passiflora ringspot potyvirus, Passiflora South African potyvirus, Passionfruit woodiness potyvirus, Patchouli mosaic potyvirus, Pea mosaic potyvirus, Pea seed-borne mosaic potyvirus, Peanut green mosaic potyvirus, Peanut mottle potyvirus, Pepper Indian mottle potyvirus, Pepper mottle potyvirus, Pepper severe mosaic potyvirus, Pepper veinal mottle potyvirus, Plum pox potyvirus, Pokeweed mosaic potyvirus, Potato A potyvirus, Potato V potyvirus, Potato Y potyvirus, Primula mosaic potyvirus, Ranunculus mottle potyvirus, Sorghum mosaic potyvirus, Soybean mosaic potyvirus, Statice Y potyvirus, Sugarcane mosaic potyvirus, Sweet potato feathery mottle potyvirus, Sweet potato G potyvirus, Swordbean distortion mosaic potyvirus, Tamarillo mosaic potyvirus, Telfairia mosaic potyvirus, Tobacco etch potyvirus, Tobacco vein-banding mosaic potyvirus, Tobacco vein mottling potyvirus, Tobacco wilt potyvirus, Tomato Peru potyvirus, Tradescantia-Zebrina potyvirus, Tropaeolum 1 potyvirus, Tropaeolum 2 potyvirus, Tuberose potyvirus, Tulip band-breaking potyvirus, Tulip breaking potyvirus, Tulip chlorotic blotch potyvirus, Turnip mosaic potyvirus, Ullucus mosaic potyvirus, Vallota mosaic potyvirus, Vanilla mosaic potyvirus, Vanilla necrosis potyvirus, Voandzeia distortion mosaic potyvirus, Watermelon mosaic 1 potyvirus, Watermelon mosaic 2 potyvirus, Wild potato mosaic potyvirus, Wisteria vein mosaic potyvirus, Yam mosaic potyvirus, Zucchini yellow fleck potyvirus, Zucchini yellow mosaic potyvirus, and the following provisional species: Asystasia gangetica mottle (?) potyvirus, Celery latent (?) potyvirus, Datura mosaic (?) potyvirus, Endive necrotic mosaic (?) potyvirus, Kalanchoe mosaic (?) potyvirus, Konjak mosaic (?) potyvirus, Nasturtium mosaic (?) potyvirus, Patchouli mottle (?) potyvirus, Shallot yellow stripe (?) potyvirus, Sweet potato vein mosaic (?) potyvirus, Welsh onion yellow stripe (?) potyvirus.*

The viral genome of potyviruses is composed of a simple RNA molecule of positive polarity that encodes a large polyprotein, which is post-translationally processed in at least 10 mature proteins by three viral proteases. This viral RNA is polyadenylated at the 3'-end; and, unlike mRNAs, it does not present a CAP structure at the 5'-end, but, instead, it is substituted by a protein encoded by the virus called VPg ("virus protein linked to the genome"), which covalently binds to the 5'-end (Murphy et al., Virology. Vol. 178, pp. 285-288, 1990).

The fact that the lack of AtDBP1 translates into a reduction in the accumulation of said factor at the post-transcriptional level seems to indicate that AtDBP1 may be exerting a control on the translation initiation factor. To this end, we decided to verify whether both proteins were capable of physically interacting. In this invention, said interaction is demonstrated; therefore, it is very likely that this factor appears as a potential candidate for the AtDBP1-mediated dephosphorylation, thanks to the type-"C protein phosphatase activity associated therewith.

In mammals, three mechanisms for the regulation of the activity of eIF4E are known. Significant amongst these is the regulation of the transcription level of this gene; in this case, the MYC proto-oncogene binds to the promoter of this factor, thereby inducing the expression levels thereof. There is also a post-translational modification via phosphorylation through two kinases (Mnk1 and 2). This phosphorylation of eIF4E seems to increase the translation, but the phosphorylated form has a lower affinity for the CAP structure; consequently, we propose that the phosphorylation could take place following the formation of the 43S pre-initiation complex, thereby promoting the release of eIF4E and the subsequent "scanning" of the ribosome. Finally, the main mechanism for the regulation of the activity of eIF4E is through the interaction thereof with a family of repressive proteins called 4E-BPs (4E-binding proteins). The binding of these proteins to eIF4E does not alter the binding to the CAP structure, but prevents the interaction of eIF4E with eIF4G, thereby suppressing the formation of the eIF4F complex. The interaction of 4E-BPs with eIF4E is controlled by the specific phosphorylation of serine and threonine residues in 4E-BP. The net effect of this phosphorylation of 4E-BP is the release of eIF4E, which makes it possible for eIF4E to actively bind to eIF4G in order to form the eIF4F complex and for the translation to proceed in a normal manner.

This invention establishes the functional involvement of AtDBP1, a transcriptional regulator with protein phosphatase activity from the DBP family, in plant-potyvirus interactions as a factor required by the virus for the efficient replication and/or propagation thereof in the plant, and its use as a modulator of the defensive response. On the basis thereof, we show that the inhibition or absence of the expression of the *AtDBP1* gene (Seq. Id. No. 9) and, therefore, the absence of the AtDBP1 factor or protein makes it possible to obtain plants with a higher resistance to potyvirus infections.

In order to determine the function of AtDBP1, plants were generated with a loss of function of the *AtDBP1* gene (Seq. Id. No. 9). To this end, homozygotic plants were selected from a line with T-DNA inserted in the AtDBP1 structural gene (SALK_005240; Seq. Id. No. 10), which carries an insertion in the *AtDBP1* gene that interrupts its encoding sequence and, consequently, must eliminate the expression thereof. This homozygotic line was called 8.1 line. The T-DNA insertion is located on the second exon of the sequence that encodes *AtDBP1* (Fig. 1A), interrupting the N-terminal region of the protein at the DNC motif, which is involved in the specific binding to DNA. Consequently, in the event that there is expression of the *AtDBP1* gene in the 8.1 plants, the resulting protein would not be functional, since it would lack the phosphatase domain and the DNA-binding capacity. In order to verify to what extent the T-DNA insertion affected the expression of the *AtDBP1* gene, the accumulation of the corresponding mRNA was analysed by means of RT-PCR in both Col-0 control plants and plants from the 8.1 line. The result of said analyses shows the absence of mRNA corresponding to the *AtDBP1* gene in the 8.1 line (Fig. 1 B). Therefore, these results confirm that the expression of AtDBP1 in 8.1 plants is inhibited as compared to the expression levels observed for the AtDBP1 gene in wild Col-0 plants.

### The inhibition of the expression of AtDBP1 triggers a lower accumulation of the eIF(iso)4E protein in the plant.

As a part of the functional, molecular and genetic characterisation of the functional homologue of DBP1 in *Arabidopsis thaliana,* and in order to identify the target genes of AtDBP1, a comparative analysis was performed between the proteome of the seedlings of the 8.1 mutant line and the seedlings of the wild Col-0 line by means of two-dimensional electrophoresis. According to this analysis, amongst the differential spots observed between both genotypes, a polypeptide was identified which, following a mass-spectroscopy analysis of the fragments thereof by digestion with trypsin, was found to correspond to one of the isoforms of translation initiation factor 4E (eIF(iso)4E), which exhibited a low accumulation of the 8.1 mutant line in the seedlings.

Factors 4E and iso4E are a part of the 4F and iso4F complexes, respectively, the latter being exclusive of plants (Browning, Plant Mol. Biol. Vol. 32, pp. 107-144, 1996). These complexes, formed by factors 4A, 4G, 4E/iso4E, and factors PABP, are required, jointly with other initiation factors, for the initiation of the translation of proteins, allowing for the binding of mRNA to the pre-initiation complex. Specifically, translation initiation factor 4E is responsible for recognising and binding to the CAP structures present at the 5'-end of mRNAs.

The low representation of factor eIF(iso)4E in the 8.1 mutant seems to indicate that AtDBP1 positively contributes to the accumulation of eIF(iso)4E. In order to verify whether the control exerted by AtDBP1 on said factor is at the transcriptional or the post-transcriptional level, the expression of eIF(iso)4E was analysed at the mRNA level by means of RT-PCR and, at the protein level, by means of Western-blot, using a specific polyclonal antibody. The results obtained indicate a lower accumulation of the eIF(iso)4E protein in the 8.1 mutant line as compared to Col-0 plants (Fig. 2A), whereas the level of mRNA, analysed by RT-PCR, is similar in both genotypes (Fig. 2B). Therefore, AtDBP1 must affect the expression of *eIF(iso)4E* at the post-transcriptional level, which suggests a possible direct interaction between both proteins.

### AtDBP1 and eIF(iso)4E interact in vivo

In order to verify whether AtDBP1 is capable of interacting with eIF(iso)4E, a two-hybrid assay was performed using translational fusions of both proteins to the DNA-binding and transcription activation domains, respectively, of the yeast activator GAL4. The expression of AtDBP1, jointly with eIF(iso)4E in yeast, was capable of inducing the expression of the *HIS3* marker gene, thereby confirming the specific interaction between both proteins (Fig. 3A). A point mutation in the C-terminal domain of AtDBP1 that reduces the protein phosphatase activity thereof significantly weakened the interaction, which suggests that the isoform of translation initiation factor 4E could be a substrate susceptible to AtDBP1-mediated dephosphorylation.

The interaction observed between AtDBP1 and eIF(iso)4E was confirmed by means of co-immunoprecipitation. Using a specific antibody against eIF(iso)4E, the presence of said factor was detected by means of Western blot in the immunoprecipitate obtained with anti-HA monoclonal antibodies from transgenic plant extracts that expressed AtDBP1 fused to the HA epitope under the control of the cauliflower mosaic virus (CaMV) 35S promoter, which is constitutively expressed (Fig. 3B). This shows that both proteins are capable of interacting in the plant *in vivo.*

### The inhibition of the expression of AtDBP1 leads to a loss of susceptibility or increased resistance to potyvirus infection

We attempted to determine whether the 8.1 mutants show specific recessive resistance to potyviruses. Western-blot analyses revealed that these mutants present lower levels of eIF(iso)4E; from this, we may conclude that the loss of function of this factor confers potyvirus resistance. Although a total absence of the eIF(iso)4E protein was not observed in the 8.1 T-DNA insertion line, the lower accumulation of eIF(iso)4E in these plants, as compared to Col-0 plants, suggests the possibility that the absence of AtDBP1 (or loss of function) produces a phenotype with potyvirus resistance. For this reason, Col-0 plants and plants from the 8.1 line were inoculated with an infectious clone of the Plum Pox Virus or sharka virus (PPV), a member of the potyvirus family. Moreover, the viral clone used was a carrier of the GFP reporter gene, which allowed for an *in vivo* follow-up of the degree of the colonisation of the viral infection. Initially, the accumulation of viral RNA in non-inoculated systemic tissue was analysed by means of quantitative RT-PCR, using specific primers for the GFP marker gene. As shown in figure 4A, fifteen days after inoculation (dpi), a marked decrease in the accumulation of GFP mRNA is observed in 8.1 plants as compared to Col-0 plants. This result correlates with the level of GFP protein detected by means of Western-blot in systemic tissue from both genotypes, where a lower accumulation of the protein is also observed in 8.1 plants as compared to Col-0 plants at 15 dpi (Fig. 4B). In turn, the GFP marker gene makes it possible to perform a follow-up of the movement and distribution of the virus by means of fluorescence microscopy. This demonstrated a significant delay in viral movement in 8.1 plants as compared to Col-0 plants, where both the viral accumulation in the vascular tissue and the movement of the virus through the petiole toward distal tissues takes place earlier on (Fig. 4C). Furthermore, for each of the times analysed following inoculation, it is observed that the non-inoculated systemic tissue analysed in Col-0 plants exhibits a greater viral invasion, which reaches almost the entire leaf, whereas in the equivalent tissue of 8.1 plants the dispersion of the virus predominantly affected the vascular tissue. Therefore, the loss of expression and, consequently, the loss of function of *AtDBP1* exhibited by the 8.1 mutant slow down and attenuate the viral infection.

Due to the absence of symptoms in infections by PPV, the response of 8.1 plants to another member of the potyvirus family that causes symptoms in *Arabidopsis,* the *Turnip Mosaic Virus* (TuMV), was also analysed. This would make it possible to determine whether the loss of susceptibility observed for PPV could be extended to other members of the family. As shown in figure 5, inoculation with TuMV caused a much less severe symptomatology in 8.1 plants than in control Col-0 plants. The symptomatology shown in figure 5 could be specified by means of visual analysis only in the leaves, since an induction of chlorosis and subsequent necrosis, which leads to the collapse of foliar tissue due to infection of the tissue by the virus, is generated in the wild plant, but not in the 8.1 plant, given its special resistance. Ultimately, said chlorosis and necrosis, in the case of severe infections or high titres of viruses, extends to the entire foliar tissue and eventually leads to general plant collapse; this collapse and death of the plant are characteristic of pathogenic infections as severe as those studied in this case. Therefore, the manipulation of the function of *AtDBP1* seems to trigger an increase in resistance or a loss of susceptibility to several members of the potyvirus family.

### DETAILED EXPLANATION OF AN EMBODIMENT EXAMPLE

### Growth of the plants and method of viral inoculation

The vegetable materials used were *Arabidopsis thaliana* (L.) Heynh plants of the Col-0 ecotype and the 8.1 mutant line, also in a Col-0 genetic background, which corresponds to the SALK_005240 T-DNA insertion line from the SALK Institute (La Jolla, USA). The seeds from these plants were stratified for 3 days at 4ºC following imbibition. Subsequently, the plants were grown in Jiffy-7 compacted substrate (Clause-Tezier Ibérica, Valencia, Spain) at 23ºC, with a photoperiod of 10 hours of light and 14 hours in the dark. The inoculum used consisted of a suspension of *Agrobacterium tumefaciens* carrying an infectious clone of the PPV virus (Plum Pox Virus or sharka virus) with the GFP marker gene. The bacterial culture was resuspended in 10 mM MES buffer, pH 5.6, 10 mM Mg₂Cl, 150 µM acetosyringone, at an optical density of 0.5 at 600 nm, and kept at ambient temperature for several hours. Five-week-old plants were inoculated with 20 µl of said inoculum, by infiltrating the distal half of one leaf per plant through the back part of the leaf. Subsequently, the inoculated plants were kept under the same light and temperature conditions in order to follow up the viral infection at different times.

### RNA extraction and purification

For the RNA extractions, TriZol (Invitrogen) was used, following the manufacturer's recommendations. The quality and integrity of the extracted RNA was analysed by means of spectrophotometry and agarose gel electrophoresis.

### Analysis of the gene expression by RT-PCR

In order to evaluate the degree of expression of certain genes, the semi-quantitative RT-PCR technique was used. The corresponding cDNA was obtained from the total RNA by reverse transcription with oligodT, using the "Revertaid H Minus First Strand cDNA Synthesis Kit" (Fermentas). The cDNA obtained was used as a template in PCR reactions with specific primers for the gene of interest in a PTC-100 Peltier Thermal Cycler. The final products were separated by means of agarose gel electrophoresis. The primers used (Seq. Id. Nos. 1 to 6) are shown in Table 1:

**TABLE 1**

| Gene | Primer sequence | Number of cycles | Seq. Id. No. |
|---|---|---|---|
| *AtDBP1* | Direct: 5'-GTCTGAGTTTGTTCCTGCTACG-3' | 27 | 1 |
| | | | |
| | Reverse: 5'-TACTGCTCATGGGTTTGTGGTC-3' | | 2 |
| *eIF(iso)4E* | Direct: 5'-CGTCTCAGAAGAAAACTCAACTGC-3' | 24 | 3 |
| | Reverse: 5'-CATCTTCCTCTGGCTTCACACTC-3' | | 4 |
| *eEF1*□ | Direct: 5'-GCACAGTCATTGATGCCCCA-3' | 18 | 5 |
| | Reverse: 5'-CCTCAAGAAGAGTTGGTCCCT-3' | | 6 |

### Real-time PCR

The samples were analysed in triplicate and the real-time PCR reactions were performed using Sybr Green PCR Master Mix (Applied Biosystems) in an ABI PRISM 7000 sequence detector. The direct and reverse primers were designed using the Primer Express computer package. The sequences of the primers used (Seq. Id. Nos. 7 and 8) are shown in Table 2:

**TABLE 2**

| Gene | Primer sequence | Seq. Id. No. |
|---|---|---|
| *GFP* | Direct: 5'-ACGTAAACGGCCACAAGTTC-3' | 7 |
| | Reverse: 5'-AAGTCGTGCTGCTTCATGTG-3' | 8 |

### Yeast two-hybrid system

In order to determine whether factor eIF(iso)4E is capable of specifically interacting with AtDBP1, the yeast two-hybrid system was used. To this end, translational fusions were generated, of AtDBP1 to the DNA-binding domain of the yeast activator GAL4 and of eIF(iso)4E to the transactivation domain of the same activator. The two fusion proteins were expressed in the PJ69-4A yeast strain and the expression of the *HIS3* marker gene was analysed, by evaluating the growth of the yeast strain transformed with both constructs in histidine-free medium supplemented with 3-amino-triazole, a competitive inhibitor of the HIS3 enzyme, as compared to control strains.

### REFERENCES

- Agrios, "Chapter 14: Plant diseases caused by viruses" (1988), Plant Pathology, 3rd Ed., San Diego, CA, Academic Press, p. 655.
- Baldi, P., Patocchi, A., Zini, E., Toller, C., Velasco, R., and Komjanc, M. (2004). Cloning and linkage mapping of resistance gene homologues in apple. Theor. Appl. Genet. 109, 231-239.
- Baulcombe, D. (2005). RNA silencing. Trends Biochem. Sci. 30, 290-293.
- Baulcombe, D. (2004). RNA silencing in plants. Nature 431, 356-363.
- Boevnik, P., and Oparka, K.J. (2005). Virus-host interactions during movement processes. Plant Physiol. 138, 1815-1821.
- Browning, K.S. (1996). The plant translational apparatus. Plant Mol. Biol. 32, 107-144.
- Carrasco, J.L., Ancillo, G., Mayda, E., and Vera, P. (2003). A novel transcription factor involved in plant defense endowed with protein phosphatase activity. EMBO J. 22, 3376-3384.
- Carrasco, J.L., Ancillo, G., Castelló, M.J., and Vera, P. (2005). A novel DNA-binding motif, hallmark of a new family of plant transcription factors. Plant Physiol. 137, 602-606.
- Carrasco, J.L., Castelló, M.J., and Vera, P. (2006). 14-3-3 mediates transcriptional regulation by modulating nucleocytoplasmic shuttling of tobacco DNA-binding protein-phosphatase-1. J. Biol. Chem. 281, 22875-22881.
- Carrington, J.C, Kasschau, K.D., Mahajan, S.K., and Schaad, M.C. (1996). Cell- to-cell and long distance transport of viruses in plants. Plant Cell 8, 1669-1681.
- Chen, M.-H., Tian, G.-W., Gafni, Y., and Citovsky, V. (2005). Effects of calreticulin on viral cell-to-cell movement. Plant Physiol. 138, 1866-1876.
- Dempsey, D.A., Shah, J., and Klessig, D.F. (1999). Salicylic acid and disease resistance in plants. Crit. Rev. Plant Sci. 18, 547-575.
- Duprat, A., Caranta, C., Revers, F., Menand, B., Browning, K.S., and Robaglia, C. (2002). The Arabidopsis eukaryotic initiation factor (iso) 4E is dispensable for plant growth but required for susceptibility to potyviruses. Plant J. 32, 927-934.
- Deng, Z., Huang, S., Ling, P., Chen, C., Yu, C., Weber, C.A., Moore, G.A., and Gmitter, F.G. (2000). Cloning and characterization of NBS-LRR class resistance-gene candidate sequences in citrus. Theor. Appl. Genet. 101, 814-822.
- Di Gaspero, G., and Cipriani, G. (2002). Resistance gene analogs are candidate markers for disease-resistance genes in grape (Vitis spp.). Theor. Appl. Genet. 106, 163-172.
- Díaz-Pendón, J.A., Truniger, V., Nieto, C., García-Mas, J., Bendahmane, A., and Aranda, M. (2004). Advances in understanding recessive resistance to plant virases. Mol. Plant Pathol. 5, 223-233.
- Dineshkumar, S.P., Whitham, S., Choi, D., Hehl, R., Corr, C., and Baker, B. (1995). Transposon tagging of tobacco mosaic virus resistance gene N: its possible role in the TMV-N-mediated signal transduction pathway. Proc. Natl. Acad. Sci. USA 92, 4175-4180.
- Dondini, L., Costa, F., Tataranni, G., Tartarini, S., and Sansavini, S. (2004). Cloning of apricot RGAs (Resistance Gene Analogs) and development of molecular markers associated with sharka (PPV) resistance. J. Horticul. Science and Biotech. 79, 729-734.
- Fraser, R.S.S. (1990). The genetics of resistance to plant viruses. Annu. Rev. Phytopathol. 28, 179-200.
- Fraser, R.S.S. (1999). Plant resistance to viruses. In: Encyclopedia of Virology (Granoff, A., and Webster, R.G., eds). Academic Press, pp. 1300-1307. San Diego, CA.
- Hagiwara, Y., Komoda, K., Yamanaka, T., Tamai, A., Meshi, T, Funada, R., Tsuchiya, T., Naito, S., and Ishikawa, M. (2003). Subcellular localization of host and viral proteins associated with tobamovirus RNA replication. EMBO J. 22, 344-353.
- Hull, R. (2002). Matthews' Plant Virology. Academic Press.
- Ishikawa, M., Naito, S., and Ohno, T. (1993) Effects of the tom1 mutation of Arabidopsis thaliana on the multiplication of Tobacco mosaic virus RNA in protoplasts. J. Virol. 67, 5328-5338.
- Ishikawa, M., Obata, F., Kumagai, T., and Ohno, T. (1991). Isolation of mutants of Arabidopsis thaliana in which accumulation of Tobacco mosaic virus coat protein is reduced to low levels. Mol. Gen. Genet. 230, 33-38.
- Kreps, J.A., Yajun, W., Chang, H.-S., Zhu, T., Wang, X., and Harper, J.F. (2002). Transcriptome changes for Arabidopsis in response to salt, osmotic, and cold stress. Plant Physiol. 130, 2129-2141.
- Lartey, R., Ghoshroy, S., and Citovsky, V. (1998). Identification of an Arabidopsis thaliana mutation (vsm1) that restricts systemic movement of tobamoviruses. Mol. Plant-Microbe Interact. 11, 706-709.
- Lellis, A.D., Kasschau, K.D., Whitham, S.A., and Carrington, J.C. (2002). Loss-of-susceptibility mutants of Arabidopsis thaliana reveal an essential role for eIF(iso)4E during potyvirus infection. Curr. Biol. 12, 1046-1051.
- Maule, A.J., Leh, V., and Lederer, C. (2002). The dialogue between viruses and hosts in compatible interactions. Curr. Opin. Plant Biol. 5, 279-284.
- Mayda, E., Marqués, C., Conejero, V., and Vera, P. (2000). Expression of a pathogen-induced gene can be mimicked by auxin insensitivity. Mol. Plant Microbe Interact. 13, 23-31.
- Murphy, J.F., Rhoads, R.E., Hunt, A.G., and Shaw, J.G. (1990). The VPg of tobacco etch virus RNA is the 49-kDa proteinase or the N-terminal 24-kDa part of the proteinase. Virology 178, 285-288.
- Nicaise, V., German-Retana, S., Sanjuan, R., Dubrana, M.P., Mazier, M., Maisonneuve, B., Candrese, T., Caranta, C., and LeGall, O. (2003). The eukaryotic translation initiation factor 4E controls lettuce susceptibility to the potyvirus Lettuce mosaic virus. Plant Physiol. 132, 1272-1282.
- Ohshima, K., Taniyama, T., Yamanaka, T., Ishikawa, M., and Naito, S. (1998). Isolation of a mutant of Arabidopsis thaliana carrying two simultaneous mutations affecting Tobacco mosaic virus multiplication within a single cell. Virology 243, 472-481.
- Revers, F., Le Gall, O., Candresse, T., and Maule, A.J. (1999). New advances in understanding the molecular biology of plant/potyvirus interactions. Mol. Plant Microbe Interact. 12, 367-376.
- Ritzenthaler, C. (2005). Resistance to plant viruses: old issue, new answers? Curr. Opin. Biotechnol. 16, 118-122.
- Robaglia, C., and Caranta, C. (2006). Translation initiation factors: a weak link in plant RNA virus infection. Trends Plant Sci. 11, 40-45.
- Ruffel, S., Dussault, M.H., Palloix, A., Moury, B., Bendahmane, A., Robaglia, C., and Caranta, C. (2002). A natural recessive resistance gene against Potato virus Y in pepper corresponds to the eukaryotic initiation factor 4E (eIF4E). Plant J. 32, 1067-1075.
- Sanford, J.C., and Johnston, S.A. (1985). The concept of parasite-derived resistance: driving resistance genes from the parasites own genome. J. Theor. Biol. 115, 395-405.
- Shen, K.A., Meyers, B.C., Islam-Faridi, M.N., Chin, D.B., Stelly, D.M., and Michelmore, R.W. (1998). Resistance gene candidates identified using PCR wiíh degenerate primers map to resistance genes clusters in lettuce. Mol. Plant Microbe Interact. 11, 815-823.
- Simón, L., Plante, D., Wittmann, S., Daigneault, N., Fortín, M.G., and Laliberté, J.-F. (2000). Complex formation between potyvirus VPg and translation eukaryotic initiation factor 4E correlates with virus infectivity. J. Virol. 74, 7730- 7737.
- Soriano, J.M., Vilanova, S., Romero, C., Yacer, G., and Badenes, M.L. (2005). Characterization and mapping of NBS-LRR resistance gene analogs in apricot (Prunus armeniaca L.). Theor. Appl. Genet. 110, 980-989.
- Stange, C. (2006). Plant-virus interactions during the infective process. Cien. Inv. Agr. 33, 1-18.
- Tsujimoto, Y., Numaga, T., Ohshima, K., Yano, M., Ohsawa, R., Goto, D., Naito, S., and Ishikawa, M. (2003). Arabidopsis tobamovirus multiplication (TOM) 2 locus encodes a transmembrane protein that interacts with TOM1. EMBO J. 22, 335-343.
- Vance, V., and Vaucheret, H. (2001). RNA silencing in plants-defense and counterdefense. Science 292, 2277-2280.
- Whitham, S., Dinesh-Kumar, S.P., Choi, D., Hehl, R., Corr, C., and Baker, B. (1994). The product of the tobacco mosaic virus resistance gene N: similarity to TOLL and the Interleukin-1 receptor. Cell 78, 1101-1115.
- Whitham, S., McCormick, S., and Baker, B. (1996). The N gene of tobacco confers resistance to tobacco mosaic virus in transgenic tomato. Proc. Natl. Acad. Sci. USA 93, 8776-8781.
- Whitham, S., Yamamoto, M., and Carrington, J. (1999). Selectable viruses and altered susceptibility mutants in Arabidopsis thaliana. Proc. Natl. Acad. Sci. USA 96, 772-777.
- Wittmann, S., Chatel, H., Fortín, M.G., and Laliberté, J.F. (1997). Interaction of the viral protein genome linked of turnip mosaic potyvirus with the translational eukaryotic initiation factor (iso) 4E of Arabidopsis thaliana using the yeast two-hybrid system. Virology 234, 84-92.
- Yamanaka, T., Imai, T., Satoh, R., Kawashima, A., Takahashi, M., Tomita, K., Kubota, K., Meshi, T., Naito, S., and Ishikawa, M. (2002). Complete inhibition of tobamovirus multiplication by simultaneous mutations in two homologous host genes. J. Virol. 76, 2491-2497.
- Yamanaka, T., Ohta, T., Takahashi, M., Meshi, T., Schmidt, R., Dean, C., Naito, S., and Ishikawa, M. (2000). TOM1, an Arabidopsis gene required for efficient multiplication of a tobamovirus, encodes a putative transmembrane protein. Proc. Natl. Acad. Sci. USA 97, 10107-10112.
- Yoshii, M., Nishikiori, M., Tomita, K., Yoshioka, N., Kozuka, R., Naito, S., Ishikawa, M. (2004). The Arabidopsis cucumovirus multiplication 1 and 2 loci encode translation initiation factors 4E and 4G. J. Virol. 78, 6102.
- Yoshii, M., Yoshioka, N., Ishikawa, M., and Naito, S. (1998a). Isolation of an Arabidopsis thaliana mutant in which accumulation of Cucumber mosaic virus coat protein is delayed. Plant J. 13, 211-219.
- Yoshii, M., Yoshioka, N., Ishikawa, M., and Naito, S. (1998b). Isolation of an Arabidopsis thaliana mutant in which the multiplication of both Cucumber mosaic virus and Turnip crinkle virus is affected. J. Virol. 72, 8731-8737.

## Claims

1. Use of the AtDBP1 gene in vegetable species as a regulator of plants' response to potyvirus infection.

2. Use of the AtDBP1 gene as claimed in claim 1, **characterised in that** said regulation consists of conferring resistance or increased resistance to potyvirus infection as compared to wild-type plants.

3. Use of the AtDBP1 gene as claimed in claims 1-2, **characterised in that** the resistance or increased resistance to potyvirus infection is produced by the loss of function of the AtDBP1 gene.

4. Use of the AtDBP1 gene as claimed in claims 1-3, **characterised in that** the loss of function is due to the inhibition of the expression thereof.

5. Use of the AtDBP1 gene as claimed in claims 1-4, **characterised in that** the inhibition of the expression thereof is produced by the 8.1 mutation.

6. Use of the AtDBP1 gene as claimed in claims 1-5, **characterised in that** it produces a lower accumulation of eIF(iso)4e.

7. Use of the AtDBP1 gene as claimed in claims 1-6, **characterised in that** the potyvirus is the Plum Pox Virus (PPV).

8. Use of the AtDBP1 gene as claimed in claims 1-6, **characterised in that** the potyvirus is the Turnip Mosaic Virus (TuMV).

9. Use of the AtDBP1 gene as claimed in claims 1-8, to obtain transgenic plants that are resistant or exhibit higher resistance to potyvirus infection.

10. Transgenic plants that are resistant or exhibit higher resistance to potyvirus infection, obtained as claimed in claim 9, **characterised by** the loss of function of the AtDBP1 gene.

11. Genetically-modified plant as claimed in claim 10, **characterised in that** the loss of function of the AtDBP1 gene is produced by the 8.1 mutation.
